# EUROPÄISCHE PATENTSCHRIFT

(11) **EP 0 833 687 B1**
(45) Veröffentlichungstag und Bekanntmachung des Hinweises auf die Patenterteilung: **08.12.1999**
(21) Anmeldenummer: 96920768.7
(22) Anmeldetag: 01.06.1996
(51) Int. Cl.: B01D 61/04, C02F 1/44

(54) **VERFAHREN ZUR WASSERAUFBEREITUNG NACH DEM PRINZIP DER UMGEKEHRTEN OSMOSE**
METHOD FOR TREATING WATER ACCORDING TO THE PRINCIPLE OF REVERSED OSMOSIS
PROCEDE POUR LE TRAITEMENT DE L'EAU SUIVANT LE PRINCIPE DE L'OSMOSE INVERSE

(30) Priorität: 08.06.1995 DE 19520913
(43) Veröffentlichungstag der Anmeldung: 08.04.1998
(73) Patentinhaber: Schäl, Wilfried, Dr.-Ing., 61350 Bad Homburg (DE)
(72) Erfinder: Schäl, Wilfried, Dr.-Ing., 61350 Bad Homburg (DE)
(74) Vertreter: Schubert, Siegmar, Dipl.-Ing.
(86) Internationale Anmeldenummer: EP9602376
(87) Internationale Veröffentlichungsnummer: WO9641674

(56) Entgegenhaltungen:
- EP-A- 0 250 019
- EP-A- 0 592 372
- DE-A- 3 106 772
- US-A- 3 505 215
- US-A- 4 724 079
- PATENT ABSTRACTS OF JAPAN vol. 005, no. 188 (C-081), 27.November 1981 & JP,A,56 111006 (EBARA INFILCO CO LTD), 2.September 1981, & DATABASE WPI Section Ch, Week 8908 Derwent Publications Ltd., London, GB; Class J01, AN 89-059261
- PATENT ABSTRACTS OF JAPAN vol. 010, no. 262 (C-371), 6.September 1986 & JP,A,61 086988 (ISHIKAWAJIMA HARIMA HEAVY IND CO LTD), 2.Mai 1986,
- PATENT ABSTRACTS OF JAPAN vol. 009, no. 032 (C-265), 9.Februar 1985 & JP,A,59 177189 (HITACHI ZOSEN KK), 6.Oktober 1984,

## Beschreibung

Die Erfindung bezieht sich auf ein Verfahren zum betrieb einer Umkehrosmoseanlage zur Wasserreinigung nach dem Obergriff des Hauptansbruches. Solche Anlagen werden unter anderem in Verbindung mit Hämodialysegeräten eingesetzt, um zur Herstellung der Dialysierflüssigkeit ausreichend reines, möglichst keimfreies Wasser zur Verfügung zu stellen.

Das Funktionsprinzip von Umkehrosmoseanlagen besteht bekanntlich darin, daß das aufzubereitende Wasser in einem Filtermodul unter hohem Druck an der Oberfläche einer semipermeablen Membran entlanggeführt wird, wobei ein Teil des Wassers, das Reinwasser (Permeat), durch die Membran tritt und auf der anderen Seite der Membran gesammelt und den Verbrauchsstellen zugeführt wird. Der nicht durch die Membran tretende, mit Zurückgehaltenen Stoffen angereicherte Teil des Rohwassers, das Abfallwasser (Konzentrat), fließt am Ende der Strömungsstrecke des Primärraumes aus dem Membranmodul aus.

Durch Bildung von Ablagerungen auf der Membran nimmt deren Durchlässigkeit und damit der Permeatfluß im Laufe der Zeit ab. Um die Brauchbarkeit des Filtermoduls möglichst lange zu erhalten, ist es im allgemeinen notwendig, der Umkehrosmoseanlage einen Wasserenthärter vorzuschalten, der vor allem die niederschlagsbildenden Calcium- und Magnesiumsalze zuruckhält und gegen Natriumchlorid austauscht. Zur Beseitigung von gröberen Verunreinigungen des Rohwassers wird üblicherweise in die Rohwasser-Zuführungsleitung, zwischen dem Wasserenthärter und der eigentlichen Umkehrosmoseeinrichtung, zusätzlich ein Vorfilter eingefügt, im allgemeinen in Form eines sogenannten Tiefenfilters mit einer auswechselbaren Filterpatrone. Der regelmäßige Austausch der Filteipatronen verursacht jedoch erhebliche Kosten. Wenn er versäumt wird, können Betriebsstörungen die Folge sein.

Aus JP-A-56 111 006 sowie aus JP-A-61 086 988 und US-A-3 505 215 sind Wasseraufbereitungsanlagen bekannt, die Vorfilter verwenden, deren Reinigung dadurch bewirkt wird, daß sie entgegen ihrer normalen Durchströmungsrichtung mit Abfallwasser (Konzentrat) durchströmt werden. Um auch während der Reinigungsphasen den Betrieb aufrechtzuerhalten, sind die Vorfilter doppelt vorhanden, wobei abwechselnd jeweils ein Filter der Filtration dient, während das andere gereinigt wird.

Die aus US-A-4 724 079 bekannte Wasseraufbereitungsanlage mit Vorfiltern, die mit einer Filtermembran ausgestattet sind, läßt das gattungsgemäße Verfahren erkennen, bei dem die Vorfilter durch Rückspülen, hier speziell mit Permeat, gereinigt werden. Zu diesem Zweck wird den Vorfiltern Permeat über eine separate Pumpe aus einem Permeat-Speichertank zugeführt. Das Problem einer ununterbrochenen Lieferung von aufbereitetem Wasser wird durch die Benutzung von Speichertanks für Permeat gelöst, jedoch ist für die Dauer des Rückspülvorgangs die Rohwasserversorgung des eigentlichen Umkehrosmoseteils, bestehend aus Hochdruckpumpe und Umkehrosmosefilter, unterbrochen, so daß dieser Anlagenteil stillgesetzt werden muß. Da das Rückspülen nur mit Permeat erfolgt, ist der Wasserverbrauch für dessen Erzeugung entsprechend erhöht.

Eine andere Möglichkeit zur Reinigung eines als Ultrafilter ausgebildeten Vorfilters ohne Unterbrechung der Reinwassererzeugung wird in EP-A-0 250 019 gezeigt. Längs der zuflußseitigen Oberfläche der Filtermembran wird zum Zwecke der Reinigung eine turbulente Strömung erzeugt. Der hierzu gebildete Rezirkulationskreislauf mit einer Pumpe ist mit einem Auslaß versehen, aus dem ein Teil der mit Verunreinigungen angereicherten unfiltrierten Flüssigkeit abgeleitet wird. Eine Umkehr der Durchströmungsrichtung der Filtermembran findet bei diesem Verfahren nicht statt.

Der Erfindung liegt die Aufgabe zugrunde, ein Verfahren zum Betrieb einer Umkehrosmoseanlage mit Niederdruck-Vorfiltration so auszugestalten, daß das zur Vorfiltration benutzte einzige Membranfilter ohne Betriebsunterbrechung des eigentlichen Umkehrosmoseteils, d.h. des Umkehrosmosefilters und der zum Druckaufbau im Umkehrosmosefilter dienenden Pumpe, durch zeitweiliges Rückspülen, d. h. Durchströmen unter Umkehrung der normalen Durchströmungsrichtung gereinigt wird, wobei der zusätzliche Wasserverbrauch möglichst gering sein soll.

Diese Aufgabe wird dadurch gelöst, daß die Speisung der Pumpe ständig durch ein stromaufwärts von ihr angeordnetes, zugleich der Rückführung überschüssig erzeugten Permeats und eines Teils des Konzentrats dienendes, steril belüftetes Puffergefäß aufrechterhalten und die Dauer des einzelnen Rückspülvorganges durch einen den Füllstand des Puffergefäßes überwachenden Sensor begrenzt wird.

In weiterer Ausgestaltung sieht die Erfindung vor, daß das Membranfilter entsprechend seiner Filtercharakteristik als Sterilfilter wirkt, und daß bei einem Ausfall des eigentlichen Umkehrosmoseteils eine Versorgung der Verbrauchsstellen mit sterilfiltriertem Rohwasser aufrechterhalten werden kann.

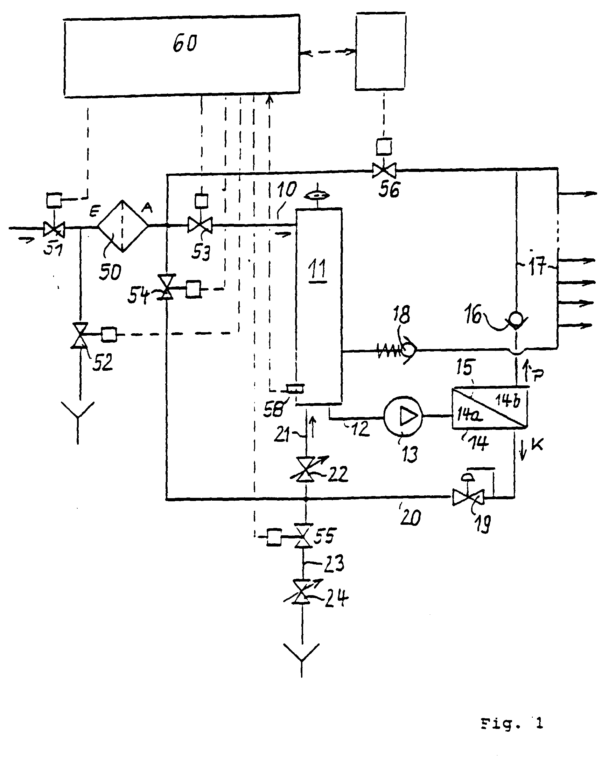

Die Abbildung zeigt das Schema einer Umkehrosmoseanlage mit Ausstattungsmerkmalen entsprechend der Erfindung. Dieses Schema ist lediglich zur Erläuterung des Funktionsprinzips gedacht, nicht zur Beschreibung technischer Einzelheiten, die dem Fachmann geläufig sind und in verschiedener Weise abgewandelt werden können. Dies betrifft z.B. den möglichen Ersatz von Teilen des Systems durch funktionell gleichwertige Einrichtungen und die Auswahl der Bauteile und die hieraus gegebenenfalls abzuleitenden technischen Maßnahmen.

Das aufzubereitende, ggf. durch einen Wasserenthärter vorbehandelte Rohwasser fließt im normalen Betrieb der Anlage über das Ventil (Magnetventil) 51, das Vorfilter 50, das Ventil 53 und die Leitung 10 in ein steril belüftetes Puffergefäß 11 mit eingebauter Füllstandsregelung. Aus dem Behälter 11 gelangt das Wasser durch die Leitung 12 über die Pumpe 13 in das Filtermodul 14, das eigentliche Umkehrosmose-Filter, dessen Primärraum 14a durch eine semipermeable Membran 15 von dem Sekundärraum 14b getrennt ist. Aus dem Sekundärraum fließt das Permeat über das Rückschlagventil 16 in die als Ringleitung ausgeführte Verbraucherleitung 17. Überschüssig erzeugtes Permeat kann am Ende der Ringleitung über ein eingefügtes Druckhalteventil 18 in das Gefäß 11 zurückfließen, wobei die Einstellung dieses Ventils den in der Verbraucherleitung 17 herrschenden Druck bestimmt.

Der für die Filtration notwendige Druck im Filtermodul 14 wird durch die Pumpe 13 in Verbindung mit einem in die Kontentratleitung 20 stromabwarts vom Filtermodul eingefügten Stromungswiderstand 19, z.B. in Form eines Druckhalteventils, hergestellt. Bei geringer Kapazitätsauslastung der Anlage und geringem Salzgehalt des Rohwassers besteht eine geeignete Maßnahme zur Wassereinsparung darin, einen Teil des Konzentrats auf die Rohwasserseite zurückzuleiten. Diesem Zweck dient die in das Puffergefäß führende Leitung 21 mit dem eingefügten Regeiventil 22. Der restliche Anteil des Konzentrats wird im normalen Betrieb über die Leitung 23 mit dem eingefügten Regelventil 24 in den Abfluß geleitet.

Die Erfindung sieht vor, daß das Vorfilter 50 rückspülbar ist und hierzu entgegen seiner normalen Fließrichtung durchstömt wird, so daß im Filtermedium oder an dessen Oberfläche angelagerte Teilchen in vorm von Schmutzpartikeln oder hochmolekularen Substanzen gelöst und mit dem Flüssigkeitsstrom in den Abfluß abgeleitet werden. Während der Dauer des Rückspülens kann sich die Anlage selbst aus dem in dem Puffergefäß 11 vorhandenen Wasservorrat versorgen, so daß eine Betriebsunterbrechung vermieden wird. Vorzugsweise erfolgt des Spülen mit Konzentrat, das normalerweise in den Abfluß geleitet würde, so daß auch kein zusätzlicher Wasserverbrauch eintritt.

Zu den genannten Zwecken steht der Ausgang des Filters 50 über das Ventil 54 mit der Konzentratleitung 20 und der Eingang des Filters 50 über das Ventil 54 fit der Abflußleitung in Verbindung. Zur Durchführung einer Rückspülung des Filters 50 werden die Ventile 51 und 53 geschlossen und die Ventile 52 und 54 geöffnet. Dadurch fließt Konzentrat von der Leitung 20 durch das Ventil 54, entgegen der normalen Strömungsrichtung durch das Filter 50 und durch das Ventil 52 in den Abfluß. Um die Durchströmung zu steigern, kann es zweckmäßig sein, in die Leitung 23, über die im normalen Betrieb die Ableitung des Kontrats in den Abfluß erfolgt, ein zusätzliches Ventil 55 einzufügen, das für die Dauer der Rückspülung geschlossen wird. Zu dem gleichen Zweck kann ein weiteres (in der Abbildung nicht gezeigtes) Ventil in die Leitung 21 eingefügt werden.

Die Steuerung des Rückspülens erfolgt durch das Steuergerat 60, das die entsprechenden Schaltsignale zu den Ventilen sendet. Dies kann mittels einer eingebauten Schaltuhr so erfolgen, daß die Rückspülung selbsttätig in bestimmten Zeitabständen ausgelöst wird. Zur Begrenzung der Dauer des Rückspülvorganges ist das Puffergefäß 11 mit einem Füllstandssensor 58 ausgestattet, der die Rückschaltung in den normalen Betrieb spätestens dann veranlaßt, wenn der Wasservorrat im Behälter annähernd verbraucht ist.

Für eine Spüldauer von einigen Sekunden wird bei Anlagen typischer Dimensionierung eine Flüssigkeitsmenge von maximal einigen Litern benötigt.

Die beschriebene Einrichtung ist für alle üblichen Arten von Vorfiltern geeignet. Im Hinblick auf die Anwendung der Umkehrosmoseanlage zur Wasserversorgung von Hämodialysegeräten ergeben sich Jedoch besondere Vorteile. Das angewandte Verfahren eröffnet die Möglichkeit, hochwertige Filter, insbesondere Sterilfilter oder Ultrafilter, die biologisches Material, wie Mikroorganismen und deren Stoffwechsel- und Zerfallsprodukte, zuruckhalten können, aber teuer und relativ empfindlich gegen Verschmutzung sind und deren Einsatz für diesen Zweck auch aus Kostengründen normalerweise nicht in Betracht kommt, als Vorfilter einzusetzen.

Obwohl die Membran des Umkehrosmosefilters 14 selbst als Sterilfilter wirkt und insofern im allgemeinen davon ausgegangen werden kann, daß die Anlage keimfreies Permeat liefert, besteht doch ein gewisses Risiko, daß sich in schlecht durchspülten Teilen der Anlage (z.B. im Umkehrosmosefilter selbst) Mikroorganismen ansiedeln. Diese Gefahr kann durch den Einsatz eines Sterilfilters oder Ultrafilters als Vorfilter erheblich vermindert werden. Auch ist eine Kontamination des Vorfilters durch den Rückspülvorgang selbst ausgeschlossen, da zum Spülen nur Wasser verwendet wird, das das gleiche Filter schon einmal passiert hat.

Darüber hinaus bietet der Einsatz eines Sterilfilters als Vorfilter die Möglichkeit eines Notbetriebes bei Betriebstörungen in anderen Teilen der Anlage, z.B. bei einem Ausfall der Pumpe 13. In diesem Falle kann die Ringleitung 17 durch ein weiteres Ventil 56 direkt mit dem Ausgang des Vorfilters 50 verbunden werden. Zwar ist bei einem solchen Notbetrieb die Funktion der Wasserentsalzung nicht mehr verfügbar, aber die Partikel- und Keimfreiheit ist bei intaktem Vorfilter nach wie vor in gleichem Maße gewährleistet. Bei Vorhandensein eines Wasserenthärters, der der gesamten Anlage üblicherweise zum Schutz des Umkehrosmosefilters vor Ausfällungen von Calcium- und Magnesiumcarbonat dient, besteht die im Wasser gelöste Substanz weit überwiegend aus Natriumchlorid. Dieser Salzgehalt kann an den von der Anlage versorgten Dialysegeräten selbst im allgemeinen durch veränderte Einstellung der Dosierung des Natriumchloridanteils des Dialysekonzentrats kompensiert werden.

Die Verbindung zwischen der Ringleitung 17 und dem Ausgang des Vorfilters 50 über das Ventil 56 bietet außerdem die Möglichkeit, das Vorfilter mit Permeat rückzuspülen. Dies kommt in Betracht, denn die Anlage zeitweise nicht oder nur zu einem Teil ihrer Kapazität ausgelastet ist.

## Patentansprüche

1. Verfahren zur Wasserreinigung nach dem Prinzip der umgekehrten Osmose, bei dem Rohwasser mittels einer Pumpe (13) unter erhöhtem Druck einem Umkehrosmosefilter (14) zugeführt und durch das Umkehrosmosefilter in filtriertes Reinwasser, sogenanntes Permeat, und mit zurückgehaltenen Stoffen angereichertes Abfallwasser, sogenanntes Konzentrat, getrennt wird, verbunden mit einer Niederdruck-Vorfiltration mittels eines stromaufwärts der Pumpe in den Rohwasserzulauf eingefügten, mit einer Filtermembran ausgestatteten Vorfilters (50), das zum Zwecke der Reinigung der Filtermembran zeitweise rückgespült, d.h. entgegen seiner normalen Filtrationsrichtung mit Flüssigkeit durchströmt wird, die einem stromabwärts vom Umkehrosmosefilter liegenden Teil der Anlage entnommen ist,
**dadurch gekennzeichnet,**
daß die Speisung der Pumpe ständig durch ein stromaufwärts von ihr angeordnetes, zugleich der Rückführung überschüssig erzeugten Permeats und eines Teils des Konzentrats dienendes, steril belüftetes Puffergefäß (11) aufrechterhalten und die Dauer des einzelnen Rückspülvorganges durch einen den Füllstand des Puffergefäßes überwachenden Sensor (58) begrenzt wird.

2. Verfahren nach Anspruch 1, dadurch gekennzeichnet, daß das Vorfilter (50) als Sterilfilter ausgebildet ist und das an dessen Ausgang (A) verfügbare sterilfiltrierte Rohwasser anstelle des Reinwassers den Verbrauchsstellen zuführbar ist.

3. Verfahren nach Anspruch 2, dadurch gekennzeichnet, daß das Rückspülen des Vorfilters mit Permeat erfolgt und die für die Zuführung sterilfiltrierten Rohwassers zu den Verbrauchsstellen dienende Leitung auch zum Rückspülen des Vorfilters mit Permeat benutzt wird.

4. Verfahren nach Anspruch 1, dadurch gekennzeichnet, daß das Rückspülen des Vorfilters wahlweise mit Konzentrat oder Permeat erfolgen kann.

## Claims

1. Process for the purification of water according to the principle of reverse osmosis, in which raw water is fed under high pressure by a pump (13) to a reverse osmosis filter(14) and is separated by the reverse osmosis filter into purified water, termed the permeate, and waste water enriched with retained substances, termed the concentrate, connected to a low-pressure preliminary filtration system comprising a preliminary filter (50) located upstream from the pump in the raw water supply line, the said filter being fitted with a filter membrane and being reverse flushed from time to time for the purpose of cleansing the said filter membrane, the said reverse flushing being carried out by passing liquid through the said filter in the direction opposite to its normal filtration direction and removing the said liquid from a part of the unit located upstream from the reverse osmosis filter,
**characterised in that**
the pump is fed continuously from a buffer vessel (11) with sterile ventilation located upstream from the pump, which serves as a return reservoir both for any excess permeate produced and for part of the concentrate, and the duration of individual reverse flushing operations is limited by a sensor (58) which monitors the filling level of the said buffer vessel.

2. Process according to Claim 1,
**characterised in that**
the preliminary filter (50) is formed as a sterile filter and sterile-filtered raw water can be supplied to the consumption points from its outlet (A) instead of the purified water.

3. Process according to Claim 2,
**characterised in that**
the preliminary filter is reverse flushed with permeate and the pipe system which supplies sterile-filtered raw water to the consumption points is also used for the reverse flushing of the preliminary filter with permeate.

4. Process according to Claim 1,
**characterised in that**
the preliminary filter can be reverse flushed with concentrate or with permeate, according to choice.

## Revendications

1. Procédé de purification de l'eau suivant le principe de l'osmose inverse, selon lequel de l'eau brute est amenée, au moyen d'une pompe (13), sous une pression accrue, à un filtre par osmose inverse (14), et est séparée par le filtre par osmose inverse en eau pure filtrée, appelée perméat, et en eau de rejet enrichie en substances retenues, appelée concentré, combiné à une filtration primaire basse pression, au moyen d'un filtre primaire (50), qui est équipé d'une membrane et est inséré dans l'alimentation en eau brute en aval de la pompe (13), et, qui, dans le but du nettoyage de la membrane du filtre, subit temporairement un lavage à contre-courant, c'est à dire est traversé dans le sens inverse de la direction normale de filtration par un écoulement de liquide prélevé d'une partie de l'installation en aval du filtre par osmose inverse, caractérisé en ce que l'alimentation de la pompe est maintenue en permanence par un réservoir tampon (11) disposé en amont de celle-ci, ventilé de manière stérile, et servant en même temps au recyclage de perméat produit on excès et d'une partie du concentré, et la durée de l'opération individuelle de levage à contre-courant étant limitée par un capteur (58) contrôlant le degré de remplissage du réservoir tampon.

2. Procédé selon la revendication 1, caractérisé en ce que le filtre primaire (50) est réalisé en tant que filtre stérile, et en ce que l'eau brute filtrée de manière stérile, disponible à sa sortie (A), peut être amenée à la place de l'eau purifiée, aux points de consommation.

3. Procédé selon la revendication 2, caractérisé en ce que le lavage à contre-courant du filtre primaire est effectué au moyen de perméat, et la conduite servant à amener de l'eau brute filtrée de manière stérile, aux points de consommation, est également utilisée pour le lavage à contre-courant du filtre primaire au moyen de perméat.

4. Procédé selon la revendication 1, caractérisé en ce que le lavage à contre-courant du filtre primaire peut être effectué, au choix, au moyen de concentré ou de perméat.
